# FASCICULE DE BREVET EUROPEEN

(11) **EP 2 443 126 B1**
(45) Date de publication et mention de la délivrance du brevet: **04.03.2015**
(21) Numéro de dépôt: 10734771.8
(22) Date de dépôt: 17.06.2010
(51) Int. Cl.: C07D 493/08, A61K 31/336, A61P 31/06

(54) **SIMALIKALACTONE E ET SON UTILISATION COMME MEDICAMENT**
SIMALIKALACTON E UND SEINE VERWENDUNG ALS MEDIKAMENT
SIMALIKALACTONE E AND USE THEREOF AS A MEDICAMENT

(30) Priorité: 18.06.2009 FR 0902959
(43) Date de publication de la demande: 25.04.2012
(73) Titulaire: Institut de Recherche pour le Développement, 13572 Marseille Cedex 02 (FR)
(72) Inventeur: JULLIAN, Valérie, F-31000 Toulouse (FR); VALENTIN, Alexis, F-31400 Toulouse (FR); DEHARO, Eric, F-40220 Tarnos (FR); BOURDY, Geneviève, F-31520 Ramonville Saint Agne (FR); HO-A-KWIE, Franciane, 31200 Toulouse (FR); CACHET, Nadia, F-38220 Saint Jean De Vaulx (FR)
(74) Mandataire: Mena, Sandra
(86) Numéro de dépôt international: PCT/FR2010/000447
(87) Numéro de publication internationale: WO 2010/146257

(56) Documents cités:
- S. BERTANI ET AL: "Simalikalactone D is responsible for the antimalarial properties of an amazonian traditional remedy made with Quassia amara L. (Simaroubaceae)" JOURNAL OF ETHNOPHARMACOLOGY, vol. 108, 2006, pages 155-157, XP002565739 cité dans la demande

## Description

L'invention a pour objet une nouvelle molécule, la Simalikalactone E, qui peut être extraite de la plante *Quassia amara,* ainsi que son utilisation comme médicament, notamment dans la prévention et le traitement du paludisme.

Le paludisme est l'une des trois maladies désignées par l'OMS comme étant l'une des plus importantes au monde, aux cotés du VIH/sida et de la tuberculose. Le paludisme tue chaque année entre 1 et 2 millions de personnes et en affecte environ 500 millions, et peut entraîner une baisse du taux de croissance allant jusqu'à 1,3% du PIB dans les pays fortement affectés. A l'origine de cette maladie se trouve un parasite du genre *Plasmodium,* transmis à l'Homme par la piqûre d'un moustique femelle du genre *Anopheles.* Il existe quatre espèces responsables du paludisme humain: *P.vivax, P.malariae, P.ovale* et *P.falciparum. P.vivax* et *P.falciparum* sont les plus courants. L'infection à *P.falciparum* est la plus sévère et peut entraîner la mort du patient au cours d'un coma fébrile. Le paludisme à *P. falciparum* est très répandu en Afrique subsaharienne où un taux de mortalité extrêmement élevé lui est en grande partie imputable. Des signes inquiétants témoignent de l'extension du paludisme à *P. falciparum* à de nouvelles régions et de sa résurgence dans des zones où il avait été éliminé ou contrôlé.

Il n'existe que peu de molécules actives contre cette maladie qui soient formulées en médicaments et l'émergence de résistances rend le problème du traitement encore plus aigu. Il est donc crucial d'identifier de nouvelles molécules ayant une activité antipaludique.

Parmi les molécules actuellement connues pour leur efficacité contre le paludisme on peut citer la quinine, ses dérivés et d'autres composés : la chloroquine, l'amodiaquine, la méfloquine, l'halofantrine, la luméfantrine, ainsi que l'artémisinine et ses dérivés tels que l'artésunate et l'artééther ou l'artéméther. Les plus connues sont issues de plantes médicinales traditionnellement utilisées dans leur pays d'origine. La quinine est issue de *Cinchona* spp. et l'artémisinine est issue de *Artemisia annua.*

Certaines de ces molécules peuvent être utilisées soit en prophylaxie (prévention lors d'un voyage en pays endémique), soit en thérapeutique (après diagnostic d'une infection).

Le cycle de *Plasmodium* est très complexe : de façon simplifiée, après un passage dans le foie, le parasite pénètre rapidement dans les globules rouges du malade, s'y reproduit jusqu'à provoquer leur éclatement et envahit les hématies voisines. La maladie se manifeste alors comme une fièvre plus ou moins anarchique au départ, puis régulière après quelques cycles de multiplication. Certains parasites évoluent alors en gamétocyte. Ces gamétocytes permettent, d'une part la poursuite du cycle chez l'Anophèle (ils ne sont pas digérés par le moustique) et d'autre part, un brassage génétique puisqu'ils déterminent les gamètes, nécessaires à la fécondation.

Les molécules telles que la quinine et l'artémisinine agissent lors de la phase de multiplication dans les globules rouges, la phase érythrocytaire qui est la phase symptomatique. En ce qui concerne les molécules ayant une activité anti-gamétocytocide, leur intérêt réside dans la diminution potentielle du taux de transmission de la maladie (infectivité) que ces molécules pourraient induire, dans une stratégie de traitement de groupe, et aussi dans la possibilité de réduire les phénomènes d'apparition de résistance rapide liée en partie aux taux de gamétocytes circulants (démontré pour la sulfadoxine-pyrimethamine (SP), et la chloroquine (souches sensibles/vs résistantes). Malgré l'intérêt d'utiliser des traitements gamétocytocides, peu d'études ont été faites visant à détecter et rationaliser l'emploi de ce type de produits. Actuellement seulement quelques molécules sont connues pour avoir cette propriété, l'artemisinine et ses dérivés, et la primaquine. La primaquine n'est cependant pas la molécule la plus adaptée pour être utilisée ainsi car elle possède une toxicité aigue proche de son niveau d'activité.

Les médicaments antipaludiques ne sont pas dénués d'effets secondaires, et notamment l'halofantrine a été associée à des troubles cardiaques et la méfloquine peut présenter une toxicité neurologique.

Les médicaments antipaludiques ont pour la plupart une durée de vie limitée, et perdent leur efficacité à cause de l'apparition de phénomènes de résistance. Par exemple, la chloroquine, ainsi que les inhibiteurs de dihydrofolate réductase (sulfadoxine/pyriméthamine : Fansidar ®) ne sont plus actifs sur la plupart des souches plasmodiales et ne sont plus employés en monothérapie. Des résistances sont également apparues pour les dérivés de l'artémisinine, qui sont les derniers médicaments mis sur le marché. Les antipaludiques qui sont devenus inefficaces ou sont en train de perdre rapidement de leur efficacité, ont été remplacés selon les directives de l'OMS par les ACT (Artemisinin based combinaison therapy), qui associent un dérivé de l'artemisinine et une autre molécule active. Cependant l'efficacité de cette stratégie thérapeutique associant deux molécules n'en est pas moins menacée par des phénomènes de résistance à l'artemisinine.

D'autre part, même si un effort important à été fait pour rendre ces ACT abordables économiquement pour les populations concernées, leur coût reste encore élevé.

A partir d'une espèce de la famille des Simaroubaceae, *Quassia amara* (L.), utilisée en médecine traditionnelle dans tout le nord-ouest de l'Amazonie et jusqu'en Amérique centrale contre les fièvres et le paludisme, nous avons identifié une nouvelle molécule, la Simalikalactone E (SkE) qui s'est montrée active contre le paludisme.

Une enquête ethnopharmacologique et des tests biologiques avaient permis (Vigneron M. et al., Journal of ethnopharmacology, 2005, 98 (3), 351-360 ; Bertani S. et al., Journal of ethnopharmacology, 2005, 98(1-2), 45-54) d'identifier les préparations à base de feuilles matures de *Quassia amara* comme intéressantes pour le traitement du paludisme. Les tests antiparasitaires *in vitro* et *in vivo* sur souris ont confirmé l'activité de cette préparation sur le paludisme (Bertani S. et al., Journal of Ethnopharmacology, 2005, 98(1-2), 45-54). Etant donné la fréquence d'utilisation de cette espèce dans les remèdes utilisés préventivement et curativement contre le paludisme, et l'activité antiparasitaire détectée, une étude phytochimique approfondie a été entreprise afin d'identifier les composants responsables de l'activité observée.

Une molécule de la famille des quassinoïdes à activité antipaludique importante a été isolée des jeunes feuilles de cette espèce : la Simalikalactone D (Bertani S. et al., Journal of Ethnopharmacology, 2006, 108(1), 155-157). Toutefois, la faible quantité de Simalikalactone D présente dans le remède traditionnel, préparé à partir de feuilles matures de *Quassia amara,* ne permettait pas de justifier l'activité observée *in vitro* et *in vivo* pour ce remède. Aussi, on a cherché à identifier d'autres actifs responsables de l'activité de *Quassia amara* contre le paludisme. Plusieurs auteurs se sont intéressés aux quassinoïdes comme principes actifs pour le traitement du paludisme ou d'autres pathologies : Z. Guo et al., Current Médicinal Chemistry, 2005, 2, 173-190 ; Guido F ; et al., International Journal for Parasitology, 1998, 28, 635-640, Curcino Vieira I. et Braz-Filho R. Studies in Natural Products, Elsevier, 2006, 33, 433-492. Mais aucune molécule n'a été retenue pour développer un médicament.

Cependant les inventeurs ont réussi, à partir de feuilles matures de *Quassia amara,* à identifier une autre molécule de la famille des quassinoïdes, la Simalikalactone E. Ils ont pu constater que celle-ci présente une moindre toxicité que la Simalikalactone D.

La Simalikalactone E répond à la formule 1 ci-dessous :

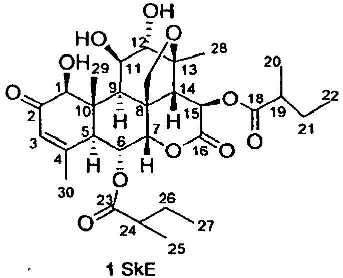

Elle constitue un premier objet de l'invention.

Cette molécule est active *in vitro* contre les souches responsables du paludisme et elle est active *in vivo* dans un modèle souris/*Plasmodium vinckei petteri* par voie orale. Elle peut donc être utilisée dans le traitement du paludisme.

Le nouveau quassinoïde, la simalikalactone E (SkE) a été isolé à partir de *Quassia amara* (Simaroubaceae), plante médicinale largement utilisée en Amazonie pour le traitement du paludisme. Cette nouvelle molécule inhibe 50% de la croissance de *P. falciparum* en culture à des dosés comprises entre 24 et 68 nM, indépendamment de la sensibilité à la chloroquine de la souche testée. Elle inhibe 50% de la croissance des cellules de mammifère Vero à une dose de 6574 nM, ce qui lui confère un index de sélectivité supérieur à 100. Nous avons également montré que cette molécule réduisait le taux de gamétocytes de 50% à une concentration 7 fois inférieure à celle obtenue avec la primaquine, la molécule de référence pour cette activité. *In vivo,* sur un modèle murin de paludisme, la SkE inhibe 50% de la croissance de *P. vinckei petteri* à des doses de 0,5 et 1 mg/kg/jour par voie intrapéritonéale et par voie orale, respectivement (la chloroquine, molécule de référence, inhibe 50% de la croissance de *P. vinckei petteri* à une dose de 3mg/kg/jour par voie intrapéritonéale).

Un second objet de l'invention est donc un médicament comprenant la SkE et un support pharmaceutiquement acceptable.

Dans un médicament destiné au traitement et/ou à la prévention du paludisme, la Simalikalactone E peut être utilisée seule ou en association avec un autre anti-paludéen.

Un tel médicament peut être administré de façon préventive chez les personnes non atteintes par le paludisme et se trouvant dans des zones à risque de façon temporaire ou permanente. Il peut être administré de façon curative chez les personnes atteintes de paludisme, en période de crise mais aussi pendant les périodes de latence de la maladie.

L'invention a donc pour objet un médicament comprenant la SkE, avec comme indication la prévention ou le traitement du paludisme. Elle a également pour objet un médicament comprenant la SkE, avec comme indication la réduction de la transmission du paludisme.

Les formes d'administration appropriées incluent les formes administrables par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Les voies d'administration préférées sont les voies orale, rectale et injectable.

On peut préparer une composition solide sous forme de comprimés, par mélange du principe actif, la SkE. avec un ou plusieurs excipients pharmaceutiques, tels que, par exemple, la gélatine, l'amidon, le lactose, le stéarate de magnésium, le talc, la silice, la gomme arabique, le mannitol, la cellulose microcristalline, l'hydroxypropyl-méthylcellulose, ou des composants analogues. On peut enrober les comprimés de saccharose, d'un dérivé cellulosique ou d'autres matières adaptées à l'enrobage. Les comprimés peuvent être réalisés par différentes techniques bien connues de l'homme du métier, telles que la compression directe, la granulation sèche, la granulation humide ou la fusion à chaud.

On peut également préparer une composition sous forme de gélules en mélangeant les ingrédients actifs avec un diluant et en versant le mélange obtenu dans des gélules molles ou dures.

Pour une administration parentérale, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Les doses journalières de SkE sont avantageusement les suivantes: de 0,01 mg/kg/j à 500 mg/kg/j de SkE. En particulier ces doses conviennent à une administration par voie orale.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient au traitement.

Le médicament de l'invention est destiné à être administré pendant préférentiellement 5 à 10 jours consécutifs, en une ou plusieurs prises quotidiennes, de préférence une seule prise par jour.

La SkE possède une activité *in vitro* équivalente, que les souches de parasite soient chloroquino-sensibles ou chloroquino-résistantes, ce qui permet d'envisager son utilisation dans des zones de paludisme chloroquino-résistant (Asie, Afrique, certaines zones d'Amérique du sud, dont Guyane).

La SkE appartient à une classe chimique différente par rapport à tous les médicaments actuellement présents sur le marché pour soigner le paludisme. Elle peut donc être efficace sur des souches résistantes aux médicaments existants.

L'activité gamétocytocide de la SkE est sept fois supérieure à celle de la primaquine, molécule de référence, qui présente en outre des problèmes de toxicité. Cette activité peut être mise à profit pour diminuer le taux de transmission et les phénomènes de résistance dans les zones ou elle serait employée.

L'activité antipaludique de plusieurs quassinoïdes, molécules de la même classe chimique que la SkE, a déjà été mise en évidence. Les quassinoïdes actifs *in vivo* sur souris impaludées sont : le sergéolide, la glaucarubinone, la cedronine, le brucéolide et ses dérivés, ainsi que la simalikalactone D (SkD).

Nous avons mesuré pour la SkE une toxicité moindre que la SkD sur des lignées cellulaire de mammifère (Cellules Vero). L'index de sélectivité de la SkE vis-à-vis de *P. falciparum* est deux fois supérieur à celui de la SkD. La SkE apporte donc un avantage par rapport à la SkD car elle est moins toxique.

Cette molécule agit aussi sur les gamétocytes, formes sexuées du parasite infectant l'Anophèle vecteur lorsqu'il aspire le sang d'un individu contaminé. Cette molécule a donc un effet sur la transmission du parasite de l'Homme à l'Anophèle (diminution de la charge sanguine en gamétocyte, entraînant une baisse de l'infectivité) et aussi sur la possible dissémination de résistances (corrélés avec le taux de gamétocytes circulant). Ce dernier point pourrait être plus particulièrement mis à profit dans des traitements associant plusieurs molécules visant à empêcher ou retarder ces phénomènes de résistance.

L'invention a encore pour objet un procédé d'isolement de la SkE à partir de feuilles de *Quassia amara.*

La SkE peut être isolée à partir de feuilles matures séchées de *Quassia amara* en employant un protocole qui comprend les étapes suivantes : les feuilles matures séchées de *Q.amara* sont broyées et extraites par du méthanol. Cet extrait est dissous dans un système biphasique à base de n-heptane, acétate d'éthyle, méthanol, eau. La phase inférieure est recueillie et son volume est réduit de moitié par évaporation sous pression réduite. Cette solution est extraite avec de l'acétate d'éthyle. L'acétate d'éthyle est évaporé. Le résidu obtenu est dissout dans du chloroforme et lavé à l'aide d'une solution aqueuse légèrement basique. La phase organique est recueillie, séchée et concentrée sous pression réduite. Cet extrait est dissout dans de l'acétate d'éthyle et lavé à l'eau. La phase organique est évaporée sous pression réduite et le résidu obtenu est élué par de l'acétate d'éthyle à travers une colonne de silice.

L'extrait obtenu est purifié suivant le protocole suivant : l'extrait est fractionné par chromatographie de partage centrifuge en utilisant un système Arizona H composé de n-heptane, acétate d'éthyle, méthanol, eau, en mode ascendant. Des échantillons de 25 mL sont recueillis. Les échantillons comprenant la SkE sont rassemblés et purifiés par chromatographie sur colonne de silice éluée par des mélanges cyclohexane/ acétate d'éthyle de polarité croissante. La SkE est éluée par le mélange cyclohexane /acétate d'éthyle 50/50.

La présente invention a également pour objet la Simalikalactone E pour la mise en oeuvre d'une méthode de traitement et/ou de prévention du paludisme qui comprend l'administration, à un patient, d'une dose thérapeutiquement efficace de SkE. Elle a encore pour objet la Simalikalactone E pour la mise en oeuvre d'une méthode de diminution de la transmission du paludisme, cette méthode comprenant l'administration à une population, de doses thérapeutiquement efficaces de SkE.

### FIGURES

Figure 1 : Tableau rapportant l'activité antiplasmodiale et la cytotoxicité de SkE.
Figure 2 : Tableau rapportant l'inhibition de la parasitémie.
Figure 3 : Durée de survie des souris (en jours) en fonction du traitement (dose) et de la voie d'administration (IP/ PO). Les traitements et les voies d'administration sont : CQ : Chloroquine (1, 5 ou 10 mg/kg/jour pendant 4 jours) SkE : Simalikalactone E (0.5, 1,5, 10 ou 20 mg/kg/jour pendant 4 jours) PO : voie orale, IP : voie intrapéritonéale.

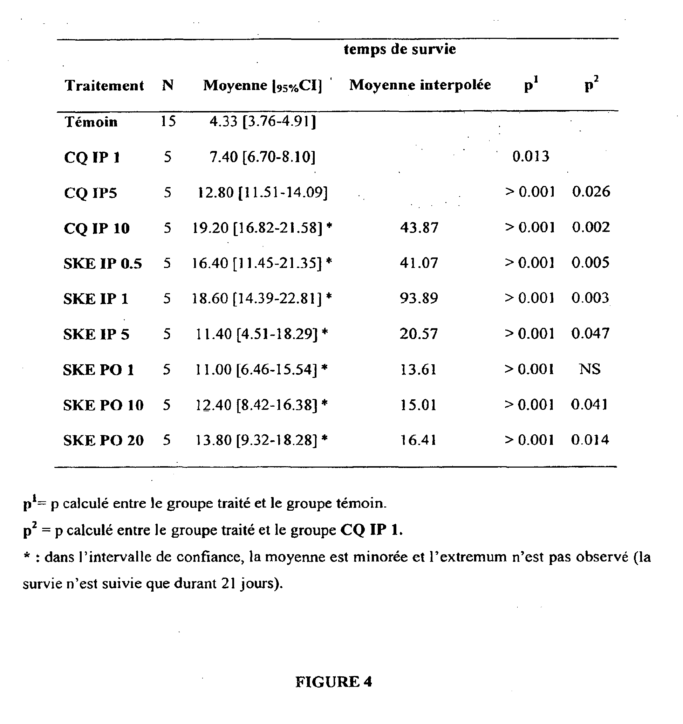

Figure 4 : Tableau rapportant la durée de survie moyenne des souris observées et calculées (interpolées) pour les différents traitements.
Figure 5 : Tableau rapportant l'activité et la cytotoxicité de la SkE sur les stades hépatiques du paludisme à *P*.*yoelii* et *P.falciparum*
Figure 6 : Représentation graphique de l'effet dose-réponse de la SkE sur les stades hépatiques de l'infection à *P. Yoelii* (A) et à *P.falciparum* (B) sur des hépatocytes primaires de rongeur ou d'humains.

### I. PARTIE EXPERIMENTALE

### A- Protocole d'obtention de la Simalikalactone E :

Les feuilles de *Quassia amara* ont été récoltées à Rémire-Montjoly en Guyane Française. Un spécimen (GB3012) a été récolté et son identification botanique a été confirmée à l'herbier de Cayenne en Guyane.

La SkE a été isolée de *Quassia amara* en employant le protocole suivant : 1 Kg de feuilles matures séchées de *Q.amara* sont broyées et extraites durant 24h avec 6 L de méthanol, ceci deux fois, pour donner 200g d'extrait. 50g de cet extrait sont dissous dans 2L d'un système biphasique composé de n-heptane, acétate d'éthyle, méthanol, eau dans les proportions 3/2/3/2. La phase la plus lourde est concentrée jusqu'à 500 mL, et 500 mL d'eau y sont ajoutés. Cette solution est extraite avec 1L d'acétate d'éthyle. L'acétate d'éthyle est évaporé sous pression réduite. Le résidu obtenu est dissout dans 1,5 L de chloroforme et lavé quatre fois à l'aide d'une solution d'hydroxyde de sodium 0,001 M. La phase organique est recueillie, séchée sur sulfate de magnésium anhydre et concentrée sous pression réduite pour donner 3,3 g d'extrait. Cet extrait est dissout dans 500 mL d'acétate d'éthyle et lavé avec 500 mL d'eau. La phase organique est évaporée sous pression réduite et le résidu obtenu est élué rapidement par l'acétate d'éthyle à travers une courte colonne de silice. On obtient après évaporation de l'acétate d'éthyle 1,0 g d'extrait. Cet extrait est fractionné par chromatographie de partage centrifuge (appareil Kromaton, équipé d'un rotor de IL), en utilisant un système Arizona H composé de n-heptane, acétate d'éthyle, méthanol, eau dans les proportions 1/3/1/3, en mode ascendant, à une température de 16°C. Le flux de solvant est de 25 mL/min, la vitesse du rotor est de 1000 t/min, et est changée au cours de l'élution pour maintenir une pression de 40 bars. L'extrait (1,0 g) est dissout dans 40 mL de système Arizona H, filtré et injecté. Des échantillons de 25 mL sont recueillis. Les échantillons 14-15-16 sont rassemblés pour donner la fraction F1 (120 mg). Cette fraction est purifiée par chromatographie sur colonne de silice éluée par des mélanges cyclohexane/ acétate d'éthyle de polarité croissante. La SkE (10 mg) est éluée par le mélange cyclohexane /acétate d'éthyle 50/50. Le rendement à partir de la plante est de 0,004 %.

La SkE a été caractérisée par spectrométrie de masse, spectroscopie RMN, infra rouge, et par son pouvoir rotatoire. Des cristaux ayant été obtenus à partir du méthanol deutéré, un cliché de rayon X de la molécule a également été obtenu. Ses caractéristiques physico-chimiques sont les suivantes :
Simalikalactone E: APCIMS, 579 (MH⁺), 561 (MH⁺-H₂O), ¹H NMR (CDCl₃, 500 MHz), δ 6.19 (m, 1H, H-15), 6.17 (s, 1H, H-3), 5.19 (dd, *J* = 2.6, 11.7 Hz, 1H, H-6), 4.75 (d, *J* = 5.1 Hz, 1H, H-11), 4.70 (d, *J* = 2.6 Hz, 1H, H-7), 4.65 (d, *J* = 7.4 Hz, 1H, H-17a), 4.19 (s, 1H, H-1), 3.83 (s, 1H, H-12), 3.70 (d, *J* = 7.4 Hz, 1H, H-17b), 3.37 (d, *J=* 11.5 Hz, 1H, H-5), 2.51 (m, 1H, H-24), 2.48 (m, 1H, H-19), 2.45 (m, 1H, H-14), 2.43 (m, 1H, H-9), 2.08 (s, 3H, H-30), 1.79 (m, 2H, H-21a, H-26a), 1.61 (m, 1H, H-26b), 1.53 (m, 1H, H-21b), 1.45 (s, 3H, H-28), 1.35 (s, 3H, H-29), 1.21 (d, *J* = 7.0 Hz, 3H, H-20), 1.19 (d, *J* = 7.0 Hz, 3H, H-25), 1.01 (t, *J* = 7.4 Hz, 3H, H-22), 0.99 (t, *J* = 7.4 Hz, 3H, H-27). ¹³C NMR (CDCl₃ 125 MHz), δ 196.5 (C-2), 176.2 (C-23), 175.2 (C-18), 166.5 (C-16), 163.0 (C-4), 126.5 (C-3), 82.8 (C-7), 81.8 (C-1), 80.0 (C-13), 79.8 (C-12), 74.2 (C-11), 70.9 (C-17), 69.1 (C-6), 67.3 (C-15), 52.7 (C-14), 50.4 (C-10), 46.1 (C-8), 45.9 (C-5), 41.3 (C-24), 41.3 (C-19), 41.1 (C-9), 27.2 (C-26), 26.7 (C-21), 26.1 (C-30), 22.8 (C-28), 16.7 (C-20), 15.6 (C-25), 12.5 (C-29), 11.7 (C-22), 11.5 (C-27). IR (KBr, cm⁻¹) : 2965, 2926, 2855, 1766, 1738, 1722,1667. [α]D²⁶ = +94° (c = 0.35, CHCl₃)

### B- Activité biologique :

### B1-Matériel et Méthodes :

### - Culture de Plasmodium falciparum :

On a utilisé les trois souches suivantes de *Plasmodium falciparum* :
- chloroquine-sensitive F32 Tanzania
- chloroquine-resistant FcB 1 Colombia
- W2 Indochina

La seule culture *in vitro* maintenue en routine est la culture des stades intra-érythrocytaires (ce qui correspond aux manifestations cliniques de la maladie). Toutes les étapes se font sous hotte à flux laminaire (PS2, Jouan) en appliquant les règles élémentaires de stérilité, afin de ne pas risquer de contamination bactérienne ou fongique de la culture qui est effectuée en absence de toute molécule antibiotique ou antifongique (ces molécules pouvant interférer avec l'évaluation des activités antiplasmodiales).

La culture de *P.falciparum* s'effectue dans des boîtes de culture stériles de 25 ou 75 cm² (fournisseur : TPP, Suisse). Chaque jour, le milieu de culture doit être changé et le pH doit rester adapté à la survie du parasite (pH ≈ 7,2-7,4). Les techniques utilisées dérivent de celles initialement décrite par Trager, W. et J.B. Jensen, Science, 1976. 193(4254): p. 673-675.

Le milieu de culture utilisé est le RPMI 1640 (fournisseur: LONZA, Emerainville).Ce milieu de départ est complémenté avec
- 25 mmol/L d'Hepes (fournisseur : LONZA, Emerainville),
- 200 mM de L-glutamine (fournisseur : LONZA, Emerainville),
- 7% v/v de sérum humain AB, (fournisseur : SHAB, Etablissement Français du Sang, Toulouse Rangueil).

On ajoute alors des globules rouges sains parasités (GRS), ainsi que des globules rouges parasités (GRP), de manière à conserver un hématocrite de 4% environ et à maintenir la parasitémie autour de 2 % (GR O⁺, Etablissement Français du Sang, Toulouse Rangueil). Pour vérifier ce point, un frottis sanguin est réalisé quotidiennement à partir de la boîte de culture et est coloré au Diff Quick (fournisseur : Dade Behring, Paris La Défense). Par lecture au microscope optique (grossissement × 100), la parasitémie est ensuite évaluée visuellement.

Si, par exemple la parasitémie après lecture du frottis est de 8 % il faut diviser le culot de culture par 4 pour ramener celle-ci à 2 %. L'hématocrite est conservé en permanence à environ 4%, c'est-à-dire de 400 µl de culot globulaire dans environ 10 ml de milieu (boîte de 25 cm²) ou 1,2 ml de culot globulaire dans environ 30 ml de milieu (boîte de 75 cm²).

### - Coloration de frottis

Le set de coloration rapide Diff Quick® donne des résultats proches de ceux obtenus par la coloration au Giemsa et est très rapide d'utilisation. Il est donc utilisé en routine pour évaluer la croissance quotidienne du parasite. La technique du Giemas, plus lourde est réservée à l'évaluation précise des parasitémies (essais de synchronisation, évaluation *in vivo*)*.*

### Diff Quick ®:

Les frottis sanguins préalablement séchés sont trempés à plusieurs reprises dans les solutions de Diff Quick® et peuvent ainsi être fixés et colorés en 30 secondes seulement.

Ce set comprend 3 réactifs:
Un fixateur : Fast Green dissout dans du méthanol (0,002g/L)
   - Un 1^{er} colorant : Eosine dissoute dans un tampon phosphate de pH = 6,6 (1,22 g/L), et 0,1 % (p/v) d'azide de sodium comme agent conservateur.
   - Un 2^{ème} colorant : colorant Thiazine dissout dans un tampon phosphate de pH = 6,6 (1,1 g/L).

### Technique d'utilisation :

Les différents réactifs doivent tout d'abord être placés dans des cuvettes de coloration à couvercle ou tout autre récipient adéquat.

Les frottis sont ensuite plongés et ressortis 5 fois de suite pendant une seconde dans le fixateur, puis 7 fois dans le 1^{er} colorant, et enfin 20 fois dans le 2^{ème}.

Un seul trempage pendant 5, 7 ou 20 secondes donne de mauvaises colorations.

L'excédent de solution doit être rapidement égoutté entre chaque réactif.

Après séchage les lames peuvent être lues au microscope optique.

### Giemsa :

Le Giemsa est un colorant neutre composé d'un colorant acide : l'éosinate azur, et de colorants basiques : le violet de méthyle, le bleu de méthyle et l'azur de méthyle, précipitant dans l'eau. Le précipité obtenu est insoluble dans l'eau mais soluble dans l'alcool méthylique.

Les éléments cellulaires acides, sont colorés sélectivement par les colorants basiques. Ces éléments sont qualifiés de basophiles (ADN parasitaire) et sont colorés en bleu-violet.

Les éléments cellulaires, basiques, sont colorés sélectivement par les colorants acides. Ces éléments sont qualifiés d'acidophiles ou d'éosinophiles (cytoplasme des hématies) et sont colorés en rose-orangé.

Les éléments neutrophiles sont colorés à la fois par les colorants acides et basiques.

### Préparation d'une solution mère de Giemsa :

A 100 ml de glycérol (fournisseur: Sigma Ultra, G6279-1L) chauffés pendant quelques minutes au bain-marie à 60°C, sont ajoutés 1,52g de Giemsa (fournisseur : Sigma, G5637-25G) dans un erlen meyer entouré d'aluminium (protection contre la lumière).

Le mélange est ensuite placé sous agitation magnétique pendant environ 4 heures à température ambiante. Du méthanol (100 ml) est ajouté et le récipient est laissé sous agitation magnétique toute la nuit.

Le lendemain l'agitation est arrêtée. La préparation est laissée au repos une semaine, puis est filtrée à l'aide de papier filtre plissé n°3 dans un flacon protégé de la lumière.

### • Préparation d' une solution mère de tampon de pH = 7,2 :

2 g de phosphate de potassium (KH₂PO₄) et 5 g de phosphate de sodium (Na₂ HPO₄, H₂O) sont ajoutés à 1 L d'eau distillée.

### • Préparation extemporanée de solution de coloration des frottis :

Cette solution est obtenue en diluant à 10 % la solution mère de Giemsa dans la solution tampon. Les frottis sont ensuite plongés à l'aide d'un portoir à lames dans la solution, et sont rincés à l'eau après environ 20 min de contact. Après séchage les lames peuvent être lues au microscope optique.

### Synchronisation de la culture par lyse des formes âgées in vitro:

Cette technique (Lambros, C. and J.P. Vanderberg, The Journal of Parasitology, 1979. 65(3): p. 418-420) est généralement réalisée sur une culture, avant d'effectuer des tests d'évaluation d'activité anti-plasmodiale *in vitro,* afin de reconstituer les conditions de développement synchrone du parasite observées *in vivo.* Elle permet d'éliminer les formes parasitaires âgées et de ne garder qu'un seul stade, les formes jeunes en anneaux. Cette technique permet aussi, associée à un enrichissement sélectif en forme âgée (Ribaut, C., et al., Malaria Journal, 2008. 7(1): p. 45), d'évaluer le stade parasitaire sur lequel agit préférentiellement la molécule testée.

Principe : Lors de sa multiplication intra-érythrocytaire, le parasite a besoin pour son métabolisme d'une grande quantité de nutriments. La cellule hôte ayant des ressources limitées, le parasite va perméabiliser la membrane érythrocytaire de manière à augmenter le trafic transmembranaire de substrats et de catabolites. La perméabilité du globule rouge augmente avec le vieillissement des parasites. La grande perméabilité aux hexitols des érythrocytes infectés par *P.falciparum* s'utilise alors pour lyser, de manière sélective, les globules rouges infectés par des parasites âgés (stade schizonte). Seules les formes parasitaires jeunes, stades anneaux, survivent.

Technique : 5g de D-sorbitol (SIGMA, France) sont solubilisés dans 100 ml d'eau distillée. Le tout est ensuite filtré par passage au travers d'un filtre millipore 0,22 µm (MILLEX GV, Cork), afin d'obtenir une solution stérile. La culture à synchroniser doit avoir une prédominance de formes jeunes (anneaux). Après centrifugation de celle-ci pendant 10 minutes, à 450 g, le surnageant est éliminé.

La solution de sorbitol précédemment préparée et chauffée à 37°C, est ajoutée goutte à goutte au culot de culture. Après homogénéisation, le tout est maintenu 10 minutes au bain-marie à 37 °C.

Puis une nouvelle centrifugation est réalisée, le surnageant contenant le sorbitol est enlevé et deux lavages du culot globulaire sont réalisés avec du RPMI sans sérum. Le culot de globules rouges est ensuite remis en culture dans une boîte contenant du milieu complet préalablement préparé et maintenu à 37 °C.

### Evaluation de l'activité antiplasmodiale:

L'activité antipaludique des différents produits est évaluée par la méthode radioactive utilisant l'hypoxanthine tritiée, décrite par Desjardins *et al*., avec les modifications apportées par Benoit et *al.* (Desjardins R.E. et al., Antimicrob. Agents Chemother, 1979, 16 : 710-718 ; F. Benoit et al. Trans. Roy. Soc. Trop. Med. Hyg. 89: 217-218).

Le test est réalisé dans des plaques de culture de 96 puits (fournisseur : TPP, Suisse) sur des cultures principalement au stade anneau. Les parasites sont mis en contact avec les produits pendant 48h à 37°C et 5 % de CO₂. La croissance du parasite est ensuite estimée par le suivi d'incorporation d'hypoxanthine tritiée dans les acides nucléiques du parasite. Les valeurs de CI₅₀ sont déterminées graphiquement sur les courbes de pourcentages d'inhibition de croissance en fonction des concentrations. Ainsi, l'activité antipaludique de la SKE a été évaluée selon le protocole suivant.

Pour chaque substance à tester, les puits sont ensemencés avec 100 µl de solution contenant les globules rouges parasités (solution à 1,5 % de parasitémie, 2% d'hématocrite) puis sont additionnés de 100 µl de dilution croissante de SKE (solution mère effectuée dans le DMSO, dilution dans le milieu de culture complet).

Une fois préparées, les plaques sont placées dans l'incubateur pendant 24 h (37°C, 5% CO₂ et atmosphère humide). La chloroquine, molécule témoin, est directement solubilisée dans du RPMI à 37 °C, additionné de 10 % de sérum.

Après 24 h d'incubation, 20 µl d'une solution d'Hypoxanthine tritiée diluée à 0,37 GBq/ml dans du RPMI, sont ajoutés dans chaque puits (Hypoxanthine tritiée 1 mCi/ml, Perkin Elmer). Les plaques sont ensuite mises à incuber pendant 24 h, puis congelées pour provoquer la lyse des globules rouges.

Les plaques sont décongelées environ une heure à température ambiante. Les globules rouges sont ensuite collectés sur un filtre (Filtre Printed Filtermat A, 1450-421, Wallac Perkin Elmer) grâce au collecteur de cellules (Filtermate Harvester, Packard). Puis le filtre est séché à température ambiante. Une fois sec, il est placé dans un sachet (Sample Bag, 1450-432, Perkin Elmer) que l'on soude, puis sont ajoutés 2 ml de liquide de scintillation (Perkin Elmer).

Le filtre est alors placé dans une cassette qui est déposée dans le compteur ß pour mesurer la radioactivité. Les résultats sont exprimés sur des courbes donnant l'inhibition de l'incorporation par rapport à un témoin ne recevant aucun principe actif (0% inhibition) en fonction de la concentration de principe actif testée. Une CI₅₀ (concentration inhibant 50% de la croissance du parasite) est alors calculée graphiquement.

### Evaluation de la cytotoxicité:

Cette évaluation a lieu sur deux types cellulaires, la lignée MCF-7 qui est une lignée de cellules tumorales mammaires et les cellules Vero (cellules de rein de singe).

Les cellules MCF-7 se cultivent dans du milieu DMEM enrichi à 10% en sérum de veau foetal (SVF). Deux fois par semaine, quand les cellules sont confluentes, il faut les repiquer (passer), c'est-à-dire les décoller de leur support afin de les reprendre pour réaliser d'autres cultures ou des tests. Le passage s'effectue en ajoutant sur le tapis cellulaire 3 ml de trypsine-EDTA (Sigma) puis en laissant incuber 3 minutes à 37°C. La trypsine-EDTA permet en digérant la matrice extra-cellulaire, de décoller les cellules adhérentes puis de les récupérer en suspension. La réaction est arrêtée par ajout de DMEM avec SVF. Le nombre de cellules est ensuite évalué sur cellule de Malassez. Une coloration vitale au bleu Trypan est effectuée afin de déterminer le pourcentage de cellules mortes (qui ne doit pas dépasser 30%).

Les cellules sont réensemencées au nombre de 3 millions par boîte de 75 cm² dans 30 ml de DMEM + 10% SVF et placées dans l'incubateur (37°C, air humidifié, 5% CO₂).

Les cellules VERO sont des cellules épithéliales de rein de singe. Elles se cultivent de la même façon que les MCF-7, mais dans du milieu EMEM complété de 10% de SVF, d'acides aminés non essentiels, de pyruvate de sodium, d'HEPES et de glutamine.

L'évaluation de la cytotoxicité suit la même méthode que le test de l'activité antipaludique, mais en remplaçant *P. falciparum* par les cellules MCF-7 ou VERO.

Cependant, il existe quelques modifications mineures, du fait du mode de culture différent. En effet, les cellules utilisées pour les tests de cytotoxicité sont des cellules adhérentes et leur croissance est plus lente. C'est pourquoi leur ensemencement en plaque 96 puits se fait 24 heures avant leur mise en contact avec les produits à tester. Après 24h, les cellules adhèrent au fond du puits, le milieu est changé, puis les molécules à tester sont ajoutées, leur dilution étant réalisée dans du milieu complet. Le milieu utilisé dépend des cellules utilisées (DMEM ou EMEM).

De même que pour le test d'activité antipaludique, l'hypoxanthine tritiée est ajoutée 24 heures après la mise en contact avec les produits et la plaque est mise au congélateur 48 heures après. Enfin la mesure de la radioactivité se fait comme pour le test d'activité antipaludique. L'évaluation des CI₅₀ se fait graphiquement comme pour celle de *P. falciparum.*

### Evaluation de l'activité gamétocytocide

L'activité gamétocytocide est évaluée uniquement sur la culture de la souche W2. La technique utilisée dérive de celle décrite par Ifediba et Vaderberg (Ifediba, T. and J.P. Vanderberg, Nature, 1981. 294 (5839): p. 364-366) avec les modifications décrites par Sall et *al.* (Sall, C., et al., Bioorganic & Médicinal Chemistry Letters, 2008. 18(16): p. 4666-4669). La culture, après lyse au D-Sorbitol, est ajustée à une parasitémie de 2% (hématocrite 4%). Les boîtes de culture (75 cm²) sont maintenues verticales avec un milieu complet contenant, en plus, 200mM d'hypoxanthine. Ce milieu est changé quotidiennement pendant 11 jours. A cette date, de la N-acétyl glucosamine est rajoutée au milieu. Deux jours plus tard, les gamétocytes sont distribués en plaque de 24 puits et les principes actifs ajoutés à concentration croissante (triplicats). Quarante huit heures plus tard, des frottis sont effectués sur chaque puits. La gamétocytémie est évaluée visuellement et une courbe donnant le pourcentage d'inhibition par rapport au témoin (puits sans principe actif) en fonction de la concentration est tracée. La CI₅₀g (gamétocyte) est alors déterminée graphiquement. Le témoin utilisé est la primaquine, antipaludique gamétocytocide de référence.

### Evaluation de l'activité antimalarique in vivo

L'activité antipaludique de la SkE a été évaluée sur des souris Swiss femelles (22 g +/- 2) infectées par *P.vinckei petteri.* Avant infection des souris ayant servies pour le test, les parasites ont été décongelés, injectés et maintenues par passage dans 2 lots de souris. Le sang parasité a été prélevé au niveau du sinus rétro-orbitaire et chaque souris de l'étude a été infectée par 2 x 10⁷ parasites par voie intra-péritonéale. Les animaux infectés ont été divisés par lots de 5 par cage et ont été traités intra-péritonéalement chaque jour pendant 4 jours. La SkE a été testée à 4 concentrations préparées dans du DMSO 90% (voie intra-péritonéale) ou de la carboxy-méthyl-cellulose (voie orale) de manière à ce que chaque souris reçoive 200 µl de préparation. L'expérience a inclut des souris parasitées témoins recevant 200 µl de DMSO 90% ou de la CMC seule par voie orale et 3 lots témoins de 5 souris chacun de ces lots étant traités par des solutions de chloroquine à 1,5 et 10 mg/kg dilués dans du PBS (voie IP).

A la fin du traitement (5 j après l'infection), un frottis sanguin a été réalisé à partir d'une goutte de sang provenant de la queue de chaque souris, fixé au méthanol et coloré au Giemsa. Les lames ont été examinées au microscope et la parasitémie comptés pour 10 000 globules rouges.

La survie des souris a été suivie pendant 21 jours.

### Évaluation de l'activité in vitro de la Simalikalactone E (SKE) contre les stades hépatiques de Plasmodium yoelii et Plasmodium falciparum :

L'activité *in vitro* de la SkE contre les stades pré-érythrocytaires a été testée sur différentes espèces de *Plasmodium.* Les sporozoïtes ont été obtenus à partir de glandes salivaires infectées de moustiques *Anopheles stephensi* nourris de culture continue de *P. falciparum* (coll. R. Sauerwein, Pays-Bas) ou de souris parasitées par *P. yoelii* (Insectarium de l'Unité INSERM 945). Les essais *in vitro* ont été réalisés en utilisant des cultures d'hépatocytes primaires humains (HH) ou de souris (MH), respectivement. Les cellules primaires ont été isolées par une perfusion de collagénase de morceaux de foie (issus de l'homme ou de souris femelles SWISS, respectivement), suivie par une purification des cellules séparées sur un gradient de percoll. Les hépatocytes ont été ensemencés dans des microplaques de 96 puits (35000 MH / puits et 82500 HH / puits) et maintenus en culture à 37 ° C et 5% de CO₂ dans un milieu de William supplémenté avec 10% de FCS (Hyclone), 100 U/mL de pénicilline, 100 mg/mL de streptomycine, 2 mM de glutamine, 5 µg/mL d'insuline et 5. 10⁻⁷ M d'hémisuccinate d'hydrocortisone. Une solution stock de SkE a été préparée dans le DMSO à 200 mg/ml puis diluée dans du milieu complet décrit ci-dessus pour obtenir les concentrations désirées. Les hépatocytes ont été infectées par des sporozoïtes de *P. yoelii* (30000) ou de *P. falciparum* (40000). Le composé SkE a été ajouté simultanément avec des sporozoïtes à différentes concentrations. Le milieu de culture a ensuite été remplacé 3h après l'inoculation des sporozoïtes, puis tous les jours. Les cultures ont été fixées 48 heures après l'infection pour *P*.*yoelii*, et 5 jours après l'infection pour *P. falciparum.* L'effet inhibiteur du composé a été quantifié, après immunomarquage fluorescent des schizontes hépatiques avec un sérum de souris dirigé contre PfHSP70 par analyse microscopique de fluorescence. L'activité a été évaluée avec le logiciel Excel pour le calcul de l'CI50, la concentration inhibant 50% de l'infection par rapport au témoin non traité.

### Evaluation de la cytotoxicité in vitro de SkE sur des hépatocytes de rongeurs ou sur des hépatocytes humains primaires.

En parallèle, la cytotoxicité du composé a été évaluée sur des hépatocytes humains et de rongeurs traités dans les mêmes conditions que dans les essais d'activité du composé à tester, par l'intermédiaire du test MTT (tétrazolium) colorimétrique (Mosmann et al, 1983). Après incubation des hépatocytes primaires de rongeur ou humains pendant 2 jours ou 5 jours, respectivement, avec les différentes concentrations du composé à tester, 100 µl de solution MTT (500µg/mL) ont été ajoutés dans chaque puits. Les plaques sont ensuite incubées pendant 4 heures à 37 ° C. Les cristaux de formazan ont ensuite été dissous dans 100 µL de mélange DMSO-éthanol (V/V). La densité optique a été mesurée par spectrophotométrie à 540 nm. Les résultats sont exprimés en pourcentage de la viabilité cellulaire par rapport au témoin non traité.

### B2-Résultats :

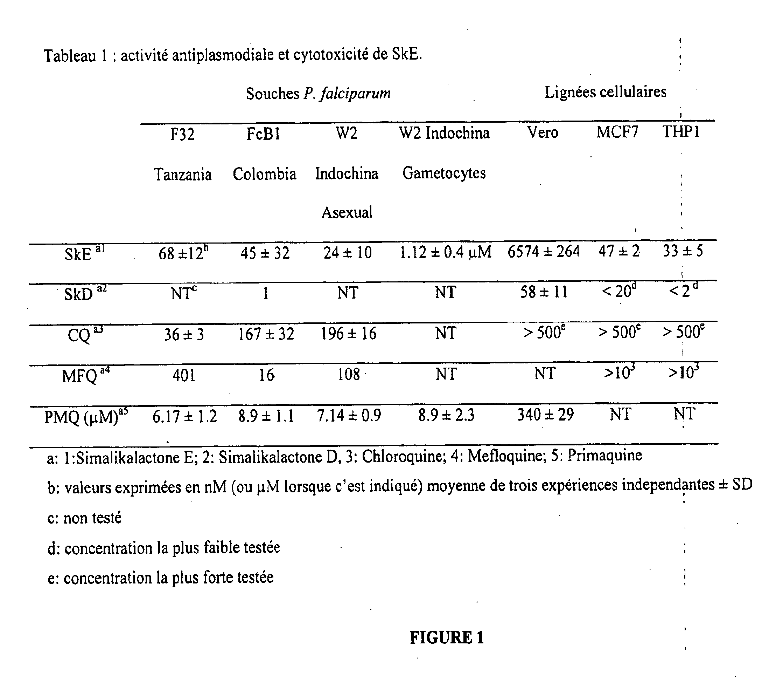

L'activité antiplasmodiale de la SkE a été déterminée *in vitro* et *in vivo.* Les résultats sont rapportés dans le tableau de la figure 1.

*In vitro* sur trois souches différentes de *P. falciparum* la SkE a des CI50 comprises entre 24 et 68 nM. L'activité ne dépend pas du niveau de chloroquinorésistance de la souche utilisée.

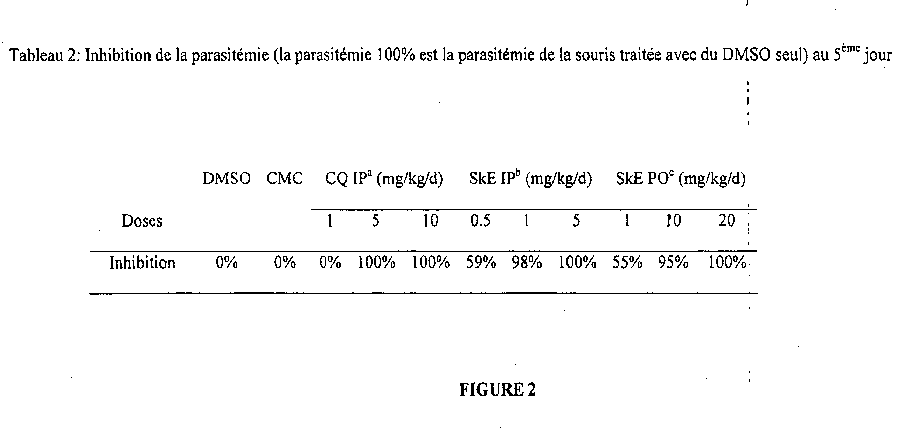

*In vivo* la SkE est active par voie orale (PO) et par voie intrapéritonéale (IP) sur des souris infectées par *P. vinckei petteri,* en suivant le protocole du test de Peters. La voie IP (Dose efficace 50, correpondant à la dose conférant une parasitémie en moyenne égale à 50% de celle des témoins : DE₅₀ = 0,5 mg/ kg/ jour) est plus efficace que la voie PO (DE₅₀ = 1 mg/ kg/ jour), le contrôle étant la chloroquine IP (DE₅₀ = 3 mg/ kg/ jour) (tableau de la figure 2).

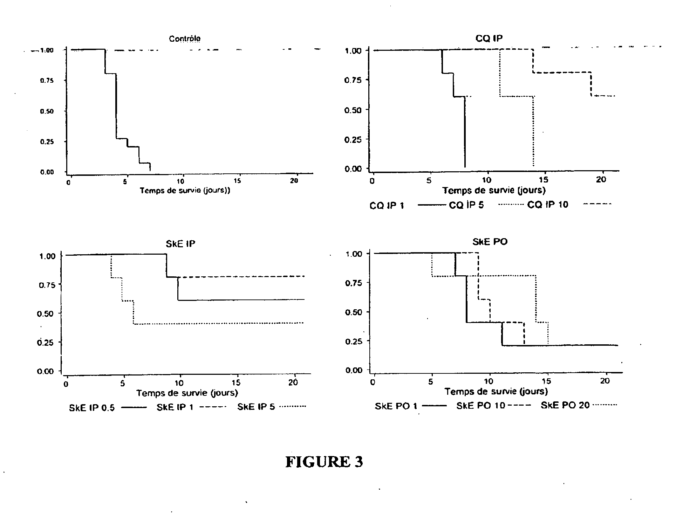

La survie des souris a été suivie pendant 3 semaines et le temps de survie est donné pour une expérimentation (Figure 3 et tableau 3). Le temps moyen de survie des souris traitées avec la SkE IP à 1mg/kg/jour est supérieur à 18,6 jours, et similaire à celui des souris traitées avec la chloroquine IP à 10 mg/kg/jour.

L'aspect des courbes de survie (Fig. 3) fait apparaître que pour la voie IP, la dose de 1 mg/kg/j est équivalente à ce qui est obtenu pour la CQ à 10 mg/kg/j, d'autre part, par voie IP, la survie des souris est toujours meilleure que celle des souris traitées à la CQ aux doses de 1 confirmé par la table 1 où p < 0,05 (hypothèse d'une différence statistiquement significative probable à 95 %). On retrouve une même différence significative quand les groupes SKE IP 0,5 et 1 sont comparés à la CQ à 5 mg/kg/j (p < 0,05).

Nous avons également mis en évidence les activités de cytotoxicité suivantes :
La cytotoxicité de la SkE dépend des cellules utilisées pour son évaluation et varie de 6 µM (Cellules Vero) à 33 nM (Cellules THP1).
La SkE agit également sur les formes sexuées du parasite (les gamétocytes), et possède une excellente activité gamétocytocide, sept fois supérieure à la primaquine, qui est la molécule de référence.

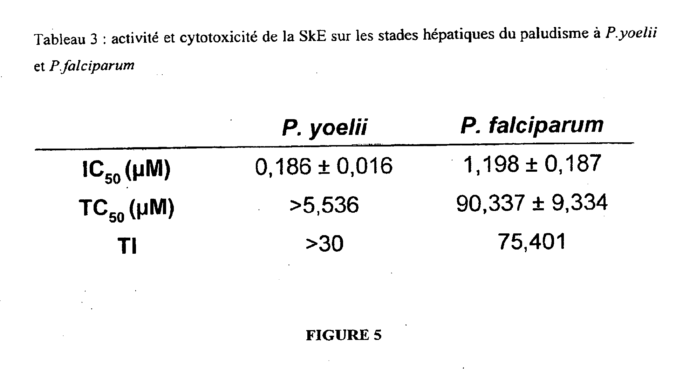

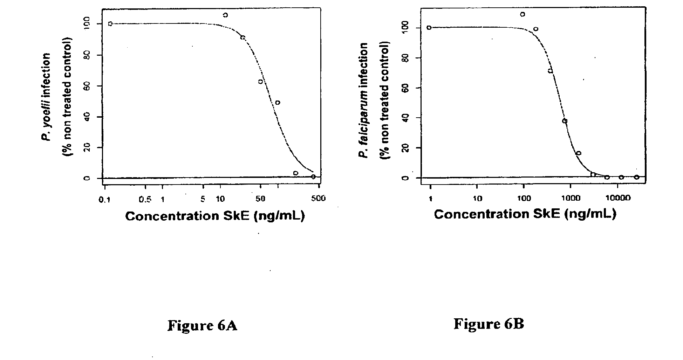

L'activité de la SkE sur les hépatocytes murins infectés par *P*.*yoelii*. Une moyenne calculée sur trois expériences a donné une CI50 de 186±16 nM (voir tableau de la figure 5 et figure 6A). Si nous comparons avec la primaquine, le médicament de référence, dans les mêmes conditions, la SkE est au moins 3 fois plus active que la primaquine (CI50 = 640nM). En parallèle, la cytotoxicité a été évaluée ; dans l'une des 3 expériences réalisées, nous avons observé une toxicité (CT50 = 1,12 µM), tandis que dans les deux autres, aucune toxicité n'a été observée avec la plus forte concentration testée (5,54 µM).
La SkE est également active contre les stades hépatiques de *P*. *falciparum* (CI50 = 1,198 ± 0.19µM moyenne calculée sur deux expériences) (tableau de la figure 5 et figure 6B). L'activité observée est inférieure à celle observée sur les plasmodia de rongeurs, mais proche de celle obtenue avec la primaquine (CI₅₀ = 0,80µM) sur la même espèce. En outre, l'index thérapeutique, calculé par la formule IT = CT₅₀/CI₅₀, (IT =75) est comparable à celui de la primaquine (IT = 81).

## Revendications

1. Molécule répondant à la formule 1 ci-dessous :

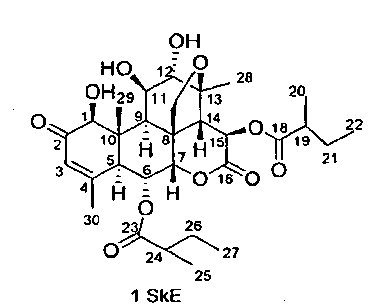

2. Médicament comprenant la molécule de formule 1 selon la revendication 1, et un support pharmaceutiquement acceptable.

3. Médicament selon la revendication 2, pour son utilisation pour la prévention et/ou le traitement du paludisme.

4. Médicament selon la revendication 2 ou la revendication 3, pour son utilisation pour réduire la transmission du paludisme.

5. Médicament selon l'une des revendications 2 à 4, qui est destiné à une administration par voie orale, rectale ou injectable ou topique.

6. Médicament selon l'une quelconque des revendications 2 à 5 dans lequel la dose journalière de SkE est de 0,01 mg/kg/j à 500 mg/kg/j de SkE.

7. Procédé d'isolement de la SkE selon la revendication 1 à partir de feuilles de *Quassia amara.*

8. Procédé selon la revendication 7 d'isolement de la SkE à partir de feuilles matures séchées de *Quassia amara* qui comprend les étapes suivantes : les feuilles matures séchées de *Q.amara* sont broyées et extraites par du méthanol, cet extrait est dissous dans un système biphasique à base de n-heptane, acétate d'éthyle, méthanol, eau, la phase inférieure est recueillie et son volume est réduit de moitié par évaporation sous pression réduite, cette solution est extraite avec de l'acétate d'éthyle, l'acétate d'éthyle est évaporé, le résidu obtenu est dissout dans du chloroforme et lavé à l'aide d'une solution aqueuse légèrement basique, la phase organique est recueillie, séchée et concentrée sous pression réduite, cet extrait est dissout dans de l'acétate d'éthyle et lavé à l'eau, la phase organique est évaporée sous pression réduite et le résidu obtenu est élué par de l'acétate d'éthyle à travers une colonne de silice.

## Patentansprüche

1. Molekül, das der untenstehenden Formel 1 entspricht:

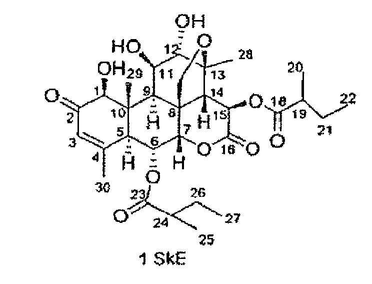

2. Medikament, das das Molekül der Formel 1 gemäß Anspruch 1 und einen pharmazeutisch annehmbaren Träger umfasst.

3. Medikament gemäß Anspruch 2 zur Verwendung für die Prävention und/oder die Behandlung von Malaria.

4. Medikament gemäß Anspruch 2 oder Anspruch 3 zur Verwendung, um die Übertragung von Malaria zu verringern.

5. Medikament gemäß einem der Ansprüche 2 bis 4, das für eine Verabreichung auf oralem, rektalem oder Injektions- oder topischem Weg bestimmt ist.

6. Medikament gemäß einem der Ansprüche 2 bis 5, wobei die tägliche Dosis von SkE 0,01 mg/kg/Tag bis 500 mg/kg/Tag SkE ist.

7. Verfahren zur Isolierung von SkE gemäß Anspruch 1 aus Blättern von *Quassia amara.*

8. Verfahren gemäß Anspruch 7 zur Isolierung von SkE aus reifen getrockneten Blättern von *Quassia amara,* das die folgenden Schritte umfasst: die reifen getrockneten Blätter von *Q. amara* werden zerkleinert und mit Methanol extrahiert, dieser Extrakt wird in einem zweiphasigen System auf der Basis von n-Heptan, Ethylacetat, Methanol, Wasser gelöst, die untere Phase wird gesammelt und ihr Volumen wird durch Einengung unter reduziertem Druck auf die Hälfte verringert, diese Lösung wird mit Ethylacetat extrahiert, das Ethylacetat wird verdampft, der erhaltene Rückstand wird in Chloroform gelöst und mit Hilfe einer wässrigen leicht basischen Lösung gewaschen, die organische Phase wird gesammelt, getrocknet und unter reduziertem Druck konzentriert, dieser Extrakt wird in Ethylacetat gelöst und mit Wasser gewaschen, die organische Phase wird unter reduziertem Druck eingeengt und der erhaltene Rückstand wird mit Ethylacetat durch eine Silicasäule eluiert.

## Claims

1. Molecule corresponding to formula I below:

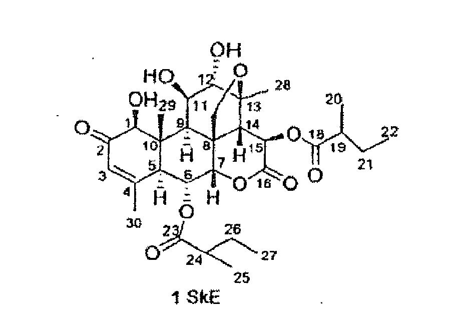

2. Medicament comprising the molecule of formula I according to claim 1 and a pharmaceutically acceptable carrier.

3. Medicament according to claim 2, for its use in the prevention and/or treatment of malaria.

4. Medicament according to claim 2 or claim 3, for its use in reducing the transmission of malaria.

5. Medicament according to any one of claims 2 to 4, which is to be administered orally, rectally, by injection or topically.

6. Medicament according to any one of claims 2 to 5, wherein the daily dose of SkE is from 0.01 mg/kg/d to 500 mg/kg/d of SkE.

7. Method for isolating SkE according to claim 1 from *Quassia amara* leaves.

8. Method according to claim 7 for isolating SkE from dried mature *Quassia amara* leaves, which method comprises the hollowing steps: the dried mature Q. *amara* leaves are ground and extracted with methanol, the extract is dissolved in a two-phase system based on n-heptane, ethyl acetate, methanol, water, the lower phase is collected and its volume is reduced by half by evaporation under reduced pressure, the solution is extracted with ethyl acetate, the ethyl acetate is evaporated off, the residue obtained is dissolved in chloroform and washed with a slightly basic aqueous solution, the organic phase is collected, dried and concentrated under reduced pressure, the extract is dissolved in ethyl acetate and washed with water, the organic phase is evaporated off under reduced pressure and the residue obtained is eluted with ethyl acetate through a silica column.
